# FASCICULE DE BREVET EUROPEEN

(11) **EP 0 949 923 B1**
(45) Date de publication et mention de la délivrance du brevet: **04.09.2002**
(21) Numéro de dépôt: 97945926.0
(22) Date de dépôt: 14.11.1997
(51) Int. Cl.: A61K 31/44, A61K 31/42, A61K 47/02

(54) **COMPOSITIONS PHARMACEUTIQUES STABILISEES, A BASE DE QUINUPRISTINE ET DE DALFOPRISTINE ET LEUR PREPARATION**
AUF QUINUPRISTIN UND DALFOPRISTIN BASIERTE STABILISIERTE PHARMAZEUTISCHE ZUSAMMENSETZUNG, UND DEREN ZUBEREITUNG
STABILISED PHARMACEUTICAL COMPOSITIONS, BASED ON QUINUPRISTINE AND DALFOPRISTINE, AND THEIR PREPARATION

(30) Priorité: 19.11.1996 FR 9614062
(43) Date de publication de la demande: 20.10.1999
(73) Titulaire: Aventis Pharma S.A., 92160 Antony (FR)
(72) Inventeur: BOUNINE, Jean-Paul, F-94700 Maisons Alfort (FR); CONRATH, Guillaume, F-92290 Châtenay Malabry (FR)
(86) Numéro de dépôt international: FR9702047
(87) Numéro de publication internationale: WO98022107

(56) Documents cités:
- EP-A- 0 135 410
- EP-A- 0 248 703
- BERNARD E. ET AL: "Pharmakokinetics and suction blister fluid penetration of a semisynthetic injectable streptogramin RP59500" EUR. J. MICROBIOL. INFECT. DIS., vol. 13, no. 9, 1994, pages 768-771, XP002037989
- 'The Merck Index, compound 4703', 1989, MERCK AND CO. INC., WHITEHOUSE STATION, NJ, USA

## Description

La présente invention concerne des compositions pharmaceutiques stabilisées destinées à l'administration parentérale de quinupristine et de dalfopristine composantes du Synercid®.

Dans la demande de brevet EP 248 703 ont été décrits les dérivés de pristinamycine I de formule générale : 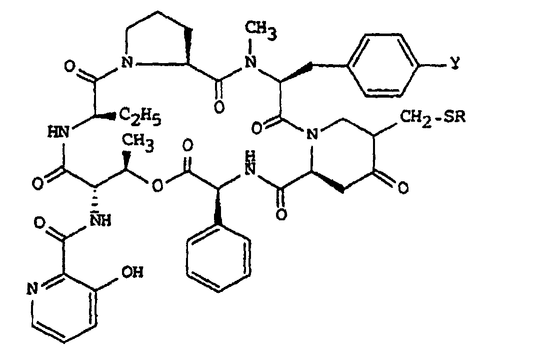 ainsi que leurs associations avec des dérivés de pristinamycine II de structure :

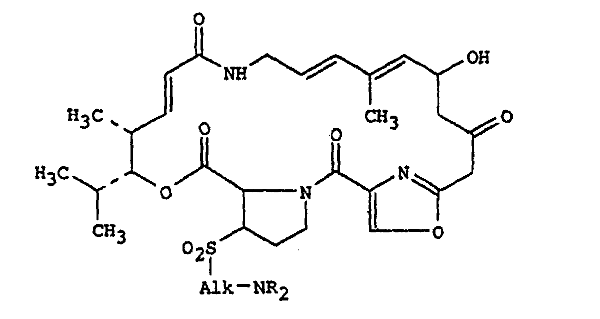

La quinupristine, dérivé de pristinamycine I, et la dalfopristine, dérivé de pristinamycine II, sont les composantes du Synercid® :

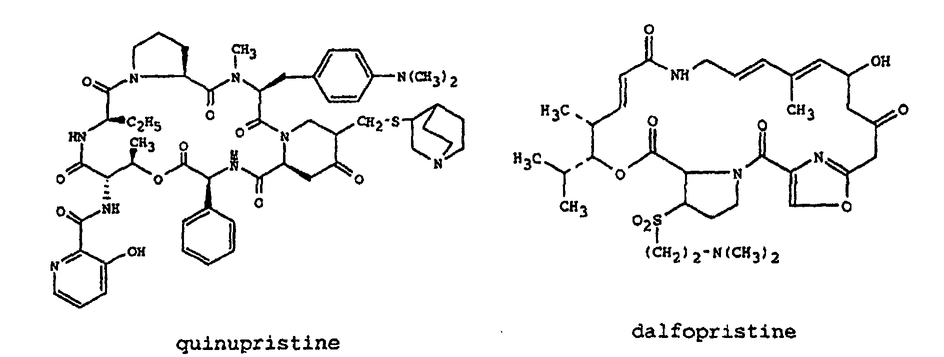

Le Synercid® (quinupristine / dalfopristine) est une combinaison 30/70, injectable, dont l'activité antibactérienne, notamment sur les germes résistants à la vancomycine, est citée dans de nombreuses publications [The Annals of Pharmacotherapy, 29, 1022-1026 (1995) ; Microbial Drug resistance, 1, 223-234 (1995)1.

La solubilisation des composantes isolées quinupristine ou dalfopristine peut être obtenue à l'état de sel.

Cependant le fait de devoir solubiliser une association pose de nombreux problèmes. Notamment le problème de trouver un agent salifiant capable de convenir à chacune des composantes. De plus, il est nécessaire de s'assurer que la composition pharmaceutique présente une stabilité telle, que le dosage de principe actif initialement fixé est constant au cours de la durée de vie du médicament. La constitution des compositions pharmaceutiques stabilisées comprenant l'association quinupristine / dalfopristine, la stabilité des solutions et/ou des lyophilisats préparés à partir des solutions, a posé de sérieux problèmes de réalisation pouvant remettre en question la possibilité d'en faire un médicament stockable et donc commercialisable. Ceci notamment du fait de l'apparition importante d'impuretés de dégradation.

Il a maintenant été trouvé que l'on pouvait stabiliser les compositions pharmaceutiques comprenant l'association quinupristine / dalfopristine, par utilisation de quantités au moins stoechiométriques d'acide méthanesulfonique ou d'acide chlorhydrique, et à un pH compris dans l'intervalle [3,5 ; 5]. Ainsi, les solutions à base d'acide méthanesulfonique ou chlorhydrique ayant un pH compris dans l'intervalle [3,5 ; 51 sont suffisament stables pour la réalisation d'une préparation industrielle et conduisent selon le cas à des lyophilisats, à des solutions reconstituées après lyophilisation et/ou à des solutions congelées stables donc stockables en vue de la commercialisation et/ou d'un usage thérapeutique.

Les compositions pharmaceutiques stabilisées selon l'invention sont notamment des solutions aqueuses comprenant :
- la dalfopristine et la quinupristine ;
- l'acide méthanesulfonique ou chlorhydrique, au moins en quantité stoechiométrique par rapport à la somme de la dalfopristine et de la quinupristine introduites ;
- si nécessaire un excès d'acide méthanesulfonique ou chlorhydrique destiné à ajuster le pH de la solution ainsi formée, dans l'intervalle [3,5 ; 5] ;
- éventuellement un agent isotonisant et/ou d'autres adjuvants pharmaceutiquement acceptables;
- une quantité d'eau convenable pour ajuster la concentration de la solution.

Les solutions ainsi obtenues présentent l'avantage d'être stabilisées.

Selon un autre aspect de l'invention elles peuvent être lyophilisées par application des techniques habituelles après abaissement de la température et élimination de l'eau. Elles peuvent être reprises par de l'eau au moment de l'injection. Selon encore un autre aspect de l'invention elles peuvent également être congelées.

Lorsque la composition obtenue est lyophilisée celle-ci peut être remise en solution au moment de l'emploi dans tout milieu injectable compatible et pharmaceutiquement acceptable. Le lyophilisat peut être avantageusement repris par exemple par de l'eau pour préparation injectable ou par des solutés de perfusion (chlorure de sodium ou solution glucosée notamment). Lorsque la solution a été congelée (poche congelée par exemple), elle peut être décongelée au moment de l'emploi. Dans une autre alternative le lyophilisat peut être aussi remis en solution diluée (contenant de préférence moins de 20 mg/ml de principe actif), ladite solution étant conservée jusqu'au moment de l'emploi.

Il est entendu que la présente invention peut s'appliquer également aux autres dérivés solubles de la pristinamycine. Par exemple elle peut aussi s'appliquer à des dérivés tels que décrits dans les brevets européens EP 133097, EP 135410, EP 191662, EP 248703.

Les compositions pharmaceutiques stabilisées selon l'invention contiennent de préférence des concentrations en principe actif quinupristine / dalfopristine comprises entre 5 et 250 mg/ml ou dans le cas d'un lyophilisat dans des proportions comprises entre 5 et 95 % ; une proportion de 20 à 90 % étant encore préférée. Il est bien entendu que des compositions pharmaceutiques stabilisées de concentration inférieure sont également réalisables et cliniquement utilisables ; ces solutions entrent également dans le cadre de la présente invention.

La quantité d'acide est fonction de la quantité de dalfopristine et de quinupristine. Elle est déterminée de manière à avoir au moins les proportions stoechiométriques et de telle manière à obtenir des solutions dont le pH est compris dans l'intervalle [3.5 ; 5].

Selon un aspect préféré de l'invention le pH de la solution initiale est fixé dans un intervalle de [3,5 ; 4,5], de préférence 3,5.

Selon un autre aspect préféré de l'invention dans le cas d'une composition lyophilisée, le pH de la solution à lyophiliser est fixé dans un intervalle de [4,5 ; 5].

Lorsque les compositions pharmaceutiques stabilisées selon l'invention contiennent un adjuvant pharmaceutiquement acceptable, ce dernier peut être choisi par exemple parmi les cosolvants, des stabilisants, des agents cryoprotecteurs, des agents dessicants, des charges et des agents isotonisants.

A titre non limitatif les cosolvants et les agents solubilisants peuvent être choisis parmi les polyéthylèneglycols (polyéthylèneglycols 300 et 400), le propylèneglycol, l'éthanol et les agents tensioactifs comme par exemple le polysorbate 80 ou les dérivés polyoxyéthylénés (crémophors) ; les charges et les agents cryoprotecteurs peuvent être notamment choisis parmi les sucres simples (par exemple le glucose, le mannitol, le fructose, le sorbitol), les disaccharides (par exemple le saccharose, le lactose, le tréhalose, le maltose) ou les polymères hydrosolubles (par exemple les dextrans, la carboxyméthylcellulose, la polyvinylpyrrolidone ou la gélatine) ; les agents stabilisants peuvent être notamment choisis parmi les agents anti-oxydants... ; les agents isotonisants peuvent être notamment choisis parmi le glucose, le chlorure de sodium, le glycérol, le sorbitol, le mannitol, le fructose, ou les dextrans 40 et 70. Lorsque le pH de la solution est élevé (pH = 5 ou voisin de 5), si l'on souhaite préparer une solution concentrée, il est préférable de choisir un agent isotonisant autre que le chlorure de sodium.

Les compositions pharmaceutiques stabilisées selon l'invention sont plus spécialement destinées à l'administration par voie parentérale. Elles peuvent être également utilisées par voie orale, occulaire, auriculaire ou en application locale sur la peau et les muqueuses.

Les compositions pharmaceutiques stabilisées selon l'invention sont préparées par mise en solution simultanée ou successive de la quinupristine, la dalfopristine, l'acide méthanesulfonique ou l'acide chlorhydrique dans de l'eau, puis ajustement du pH dans l'intervalle [3,5 ; 5] et/ou addition d'un agent isotonisant et/ou addition d'autres adjuvants pharmaceutiquement acceptables et le cas échéant lyophilisation et/ou congélation.

Selon un aspect préféré, les compositions selon l'invention sont préparées par dissolution de la composante quinupristine puis de la composante dalfopristine dans de l'eau acidifiée par l'acide méthanesulfonique ou l'acide chlorhydrique, suivie le cas échéant de l'ajustement du pH dans l'intervalle [3,5 ; 5] et/ou de l'addition d'un agent isotonisant et/ou d'autres adjuvants pharmaceutiquement acceptables. Elles sont le cas échéant lyophilisées et/ou congelées.

La préparation et la répartition de la solution sont effectuées généralement de 0°C à la température ambiante, de préférence à basse température, cette température étant fonction de la durée de la préparation et du pH. Notamment on opère à une température inférieure à 10°C.

Les compositions pharmaceutiques stabilisées selon l'invention peuvent éventuellement être stérilisées. On opère avantageusement par filtration stérilisante.

Les compositions pharmaceutiques stabilisées selon l'invention, à l'état liquide, lyophilisé ou congelé présentent l'avantage d'une stabilité physico-chimique optimale permettant une conservation pendant une durée suffisante pour en faire un médicament stockable et donc commercialisable.

Selon un aspect préféré de l'invention, on entend par compositions pharmaceutiques stabilisées, les compositions ne présentant pas plus de :
- 2,0% d'augmentation du taux de chacune des impuretés majeures de dégradation (A et B) après conservation 24 mois à 4°C ou 12 mois à 20°C lorsqu'il s'agit d'un lyophilisat,
- 2,0% d'augmentation du taux de chacune des impuretés majeures de dégradation (A et B) après conservation 18 heures entre 0 et 6°C lorsqu'il s'agit de solutions concentrées avant ou après lyophilisation,
- 2,0% d'augmentation du taux de chacune des impuretés majeures de dégradation (A et B) après conservation 3 mois à -20°C lorsqu'il s'agit d'une composition congelée,
- 5% d'augmentation du taux de chacune des impuretés majeures de dégradation (A et B) après conservation 6 heures à température ambiante ou 72 heures à 4°C lorsqu'il s'agit de solutions diluées pour perfusion.

Les exemples suivants donnés à titre non limitatif illustrent la présente invention.

### Exemple 1

On prépare un litre d'une solution à 125 mg/ml de quinupristine/dalfopristine (30/70), salifiée par de l'acide méthanesulfonique (≈ 16,7 mg/ml) à un pH de 4,75 en introduisant 810 g d'eau pour préparation injectable dans une cuve de dissolution équipée d'une station de réfrigération. La solution est réfrigérée à une température comprise entre 0 et 6°C tout au long de la fabrication. On ajoute 16,4 g d'acide méthanesulfonique, puis on introduit successivement 37,5 g de quinupristine qui sont dissous par agitation mécanique et 87,5 g de dalfopristine qui sont également dissous par agitation mécanique. Le pH de la solution est ajusté à 4,75 par une solution 1N d'acide méthanesulfonique. La solution est complétée à 1 litre (1030 g) avec de l'eau pour préparation injectable.

Cette solution est stérilisée par filtration stérilisante (filtre 0,22 µm) et répartie dans des flacons [500 mg de quinupristine/dalfopristine (30/70) par flacon] puis lyophilisée. [congélation : température -30°C à -50°C ; vitesse de congélation environ 0,5°/min. Sublimation ; pression 0,5 mbar. Dessiccation secondaire : pression (≈ 30 µbars) température 40°C].

La qualité et la stabilité au cours du stockage des lyophilisats ou de la solution reconstituée sont évaluées par une méthode de chromatographie liquide haute performance en phase inverse (CLHP) qui permet de déterminer la teneur en dalfopristine et en quinupristine et la teneur en impuretés de dégradation.

### analyse CLHP:

| | |
|---|---|
| Phase stationnaire | Silice greffée octadécylsilane, Lichrospher-100 RP18, 5µm |
| Détection | U.V. 254 nm |
| Dimensions de la colonne | 125 x 4 mm |
| Température de la colonne | 40°C |
| Phase mobile A | Tampon phosphate pH 2,9 .....80 V |
| | Acétonitrile .........................20 V |
| Phase mobile B | Tampon phosphate pH 2.9 .....35 V |
| | Acétonitrile .........................65 V |
| Gradient | de 0% phase mobile B à 66% phase mobile B en 42,5 min suivi d'un retour à 0% phase mobile B en 1,5 min et rééquilibrage pendant 5 min |
| Débit de la phase mobile | 1,1 ml/min |

La méthode d'analyse par CLHF permet de déterminer les teneurs en quinupristine et dalfopristine avec une précision de 2% et les impuretés de dégradation sont déterminées à 0,1% près.
Deux lots, lyophilisés, de quinupristine/dalfopristine (30/70) constitués de flacons de 500 mg de principe actif (lot 1a et lot 1b) ont été fabriqués selon le procédé ci-dessus et leur stabilité étudiée à 4°C au cours d'un stockage pendant une période de 2 ans. Les résultats de l'étude de stabilité de chacun de ces 2 lots montre une bonne conservation des titres des principes actifs et une dégradation peu importante (voir tableaux I et II).
Des solutions de quinupristine/dalfopristine (30/70) sont reconstituées à partir de ces lyophilisats, en reprenant par 5,0 ml de glucose à 5 %. Dans ces conditions, la stabilité de la solution concentrée ainsi constituée (prémix) étudiée sur une période de 60 minutes est jugée largement satisfaisante pour permettre une dilution ultérieure dans une poche de perfusion (voir tableau III).
Dans les conditions de dilution prévues pour une administration clinique (500 mg de quinupristine/dalfopristine (30/70) dans une poche de 250 ml de glucose à 5 %), la formulation assure une stabilité satisfaisante sur une période au moins égale à 72 heures à 4°C ou 6 heures à 25°C (voir tableaux IV et V).

### Résultats de stabilité des lyophilisats sur 2 ans :

**Tableau I**

| Etude de stabilité du lot 1a sur une période de 24 mois à 4°C | | | | | | | |
|---|---|---|---|---|---|---|---|
| Lot 1a | T0 | 3 mois | 6 mois | 9 mois | 12 mois | 18 mois | 24 mois |
| *Titre (mg*/*flacon)* | | | | | | | |
| Dalfopristine | 350 | 347 | 343 | 343 | 345 | 358 | 342 |
| Quinupristine | 149 | 146 | 147 | 149 | 145 | 153 | 144 |

| *% impuretés* | | | | | | | |
|---|---|---|---|---|---|---|---|
| (A) | 1,0 | 1,0 | 1,0 | 0,9 | 0,9 | 1,0 | 0,8 |
| (B) | 0,5 | 0,6 | 0,7 | 0,4 | 0,6 | 0,6 | 0,5 |
| (C) | 0,2 | 0,1 | 0,2 | 0,1 | 0,1 | 0,1 | 0,1 |
| (D) | 0,5 | 0,5 | 0,5 | 0,4 | 0,4 | 0,5 | 0,3 |
| (E) | 0,3 | 0,2 | - | 0,1 | 0,2 | 0,1 | 0,1 |

**Tableau II**

| Etude de stabilité du lot 1b sur une période de 24 mois à 4°C | | | | | | |
|---|---|---|---|---|---|---|
| Lot 1b | T0 | 6 mois | 9 mois | 12 mois | 18 mois | 24 mois |
| *Titre (mg*/*flacon)* | | | | | | |
| Dalfopristine | 349 | 340 | 343 | 343 | 340 | 340 |
| Quinupristine | 148 | 144 | 146 | 142 | 143 | 142 |

| *% impuretés* | | | | | | |
|---|---|---|---|---|---|---|
| (A) | 1,0 | 0,8 | 0,8 | 0,9 | 0,9 | 0,8 |
| (B) | 0,5 | 0,6 | 0,4 | 0,6 | 0.4 | 0,5 |
| (C) | 0,2 | 0,2 | 0,1 | 0,2 | 0,1 | 0,2 |
| (D) | 0,4 | 0,4 | 0,4 | 0,4 | 0,4 | 0,3 |
| (E) | 0,1 | - | 0,2 | 0,2 | 0,2 | 0,1 |

**Tableau III**

| Résultats de stabilité de la solution concentrée reconstituée à partir de lyophilisats(premix): | | | | | |
|---|---|---|---|---|---|
| Premix | T0 | 15 min | 30 min | 45 min | 60 min |
| *Titre* | | | | | |
| *(% par rapport To)* | | | | | |
| Dalfopristine | 100,0 | 99,7 | 100,2 | 99,0 | 99,0 |
| Quinupristine | 100,0 | 100,2 | 100,7 | 99,6 | 101,1 |

| *% impuretés* | | | | | |
|---|---|---|---|---|---|
| (A) | 0,74 | 0,89 | 1,02 | 1,12 | 1,28 |
| (B) | 0,51 | 0,62 | 0,69 | 0,72 | 0,78 |
| (D) | 0,47 | 0,49 | 0,46 | 0,53 | 0,61 |
| Somme des autres impuretés | 0,28 | 0,30 | 0,32 | 0,33 | 0,38 |

### Résultats de stabilité des solutions pour perfusion :

Trois lots de lyophilisats fabriqués comme décrit ci-dessus (lot 1c, lot 1d, lot 1e) ont été utilisés pour tester la stabilité des solutions diluées dans les conditions cliniques (500 mg de quinupristine/dalfopristine (30/70) lyophilisé dilué dans 250 ml de glucose à 5%).

**Tableau IV**

| Stabilité des solution diluées pour perfusion sur une période de 72 heures à 4°C | | | | | | |
|---|---|---|---|---|---|---|
| | lot 1c | | lot 1d | | lot 1e | |
| Lots testés | To | 72h à 4°C | To | 72h à 4°C | To | 72h à 4°C |
| *(mg*/*250 ml)* | | | | | | |
| Dalfopristine | 313 | 297 | 314 | 301 | 318 | 306 |
| Quinupristine | 135 | 127 | 134 | 129 | 136 | 131 |

| *% impuretés* | | | | | | |
|---|---|---|---|---|---|---|
| (A) | 1,5 | 4,2 | 1,1 | 4,1 | 1,6 | 4,5 |
| (B) | 0,7 | 0,9 | 0,6 | 0,8 | 0,8 | 1,0 |
| (C) | 0,2 | 0,3 | 0,1 | 0,3 | 0,2 | 0,3 |
| (D) | 0,3 | 0,4 | 0,4 | 0,5 | 0,4 | 0,4 |
| (E) | 0,3 | 0,3 | 0,2 | 0,2 | 0,6 | 0,3 |

**Tableau V**

| Stabilité des solution diluées pour perfusion sur une période de 6 heures à température ambiante | | | | | | |
|---|---|---|---|---|---|---|
| Lots testés | lot 1c | | lot 1d | | lot 1e | |
| | To | 6h à température ambiante | To | 6h à température ambiante | To | 6h à température ambiante |
| *(mg*/*250 ml)* | | | | | | |
| Dalfopristine | 314 | 304 | 321 | 311 | 319 | 313 |
| Quinupristine | 134 | 132 | 137 | 136 | 137 | 136 |

| *% impuretés* | | | | | | |
|---|---|---|---|---|---|---|
| (A) | 1,3 | 4,5 | 1,1 | 4,2 | 1,7 | 4,7 |
| (B) | 0,7 | 0,8 | 0,6 | 0,7 | 0,6 | 0,7 |
| (C) | 0,2 | 0,5 | 0,2 | 0,4 | 0,2 | 0,5 |
| (D) | 0,2 | 0,3 | 0,3 | 0,4 | 0,2 | 0,3 |
| (E) | 0,3 | 0,3 | 0,2 | 0,2 | 0,3 | 0,3 |

### Exemple 2

On prépare des solutions à 125 mg/ml de quinupristine/dalfopristine (30/70), à un pH de 4,50, salifiées soit par l'acide chlorhydrique soit par l'acide méthanesulfonique selon le protocole suivant:
350 g d'eau pour préparation injectable sont introduits dans un bêcher de dissolution. A température ambiante, 30,0 g de quinupristine sont dispersés dans l'eau à l'aide d'une agitation mécanique. Une solution 1N d'acide méthanesulfonique ou d'acide chlorhydrique est ajoutée jusqu'à dissolution et obtention d'un pH de 4,50. 70,0 g de dalfopristine sont dispersés dans l'eau à l'aide d'une agitation mécanique, puis additionnés d'une solution 1N d'acide méthanesulfonique ou d'acide chlorhydrique jusqu'à dissolution et obtention d'un pH de 4,50. Une homogénéisation est effectuée pendant 10 minutes.
La solution est complétée à 0,8 litre (824 g) avec de l'eau pour préparation injectable.
La lyophilisation de cette solution selon le protocole décrit dans l'exemple 1 permet d'obtenir des lyophilisats dont la stabilité en cours de stockage est satisfaisante. Une étude de stabilité en cours de stockage à 4°C et à température ambiante (TA) confirme la bonne stabilité des formulations (Cf. tableaux VI et VII).

**Tableau VI**

| Stabilité des lyophilisats à base d'acide méthanesulfonique sur une période de 1 an à 4°C et à température ambiante : | | | | | | | |
|---|---|---|---|---|---|---|---|
| | T0 | 6 mois | | 9 mois | | 1 an | |
| Acide méthane sulfonique | | 4°C | T.A. | 4°C | T.A. | 4°C | T.A. |
| *Titre (mg*/*flacon)* | | | | | | | |
| Dalfopristine | 332 | 322 | 299 | 328 | 319 | 324 | 310 |
| Quinupristine | 143 | 150 | 135 | 149 | 145 | 146 | 143 |

| *% impuretés* | | | | | | | |
|---|---|---|---|---|---|---|---|
| (A) | 3,7 | 4,0 | 3,9 | 3,4 | 3,3 | 4,3 | 4,4 |
| (B) | 1,9 | 1,7 | 1,7 | 1,6 | 2,2 | 1,9 | 2,8 |
| (C) | 0,3 | 0,5 | 0,9 | 0,3 | 0,3 | 0,3 | 0,3 |
| (D) | 1,4 | 1,4 | 1,5 | 1,2 | 1,2 | 1,4 | 1,4 |

**Tableau VII**

| Stabilité des lyophilisats à base d'acide chlorhydrique sur une période de 6 mois à 4°C et à température ambiante : | | | |
|---|---|---|---|
| | | 6 mois | |
| Acide chlorhydrique | T0 | 4°C | T.A. |
| *Titre (mg*/*flacon)* | | | |
| Dalfopristine | 327 | 333 | 313 |
| Quinupristine | 156 | 150 | 145 |

| *% impuretés* | | | |
|---|---|---|---|
| (A) | 1,7 | 1,6 | 1,4 |
| (B) | 1,6 | 1,6 | 2,4 |
| (C) | 0,3 | 0,2 | 0,2 |
| (D) | 0,9 | 0,4 | 0,4 |

### Exemple 3

On prépare respectivement selon l'exemple 1. ou 2, des solutions à 125 mg/ml de quinupristine/dalfopristine (30/70) à différents pH par addition d'une quantité variable d'acide méthanesulfonique 1N ; ces solutions sont destinées à la lyophilisation ou à la congélation. Le pH des solutions pour lyophilisation est fixé dans une gamme comprise entre 4,5 et 4,8. Le pH des solutions destinées à la congélation est fixé dans une gamme comprise entre 3,5 et 4,5.

L'étude de stabilité des lots de lyophilisats (préparés dans les conditions de dissolution des principes actifs décrites à l'exemple 1, mais à l'échelle de 600 ml, en introduisant 150 ml d'eau et 35 à 50 ml d'une solution 1N d'acide méthanesulfonique avant dispersion des principes actifs), montre sur une période de 4 ans, une stabilité optimale (voir tableau VIII).

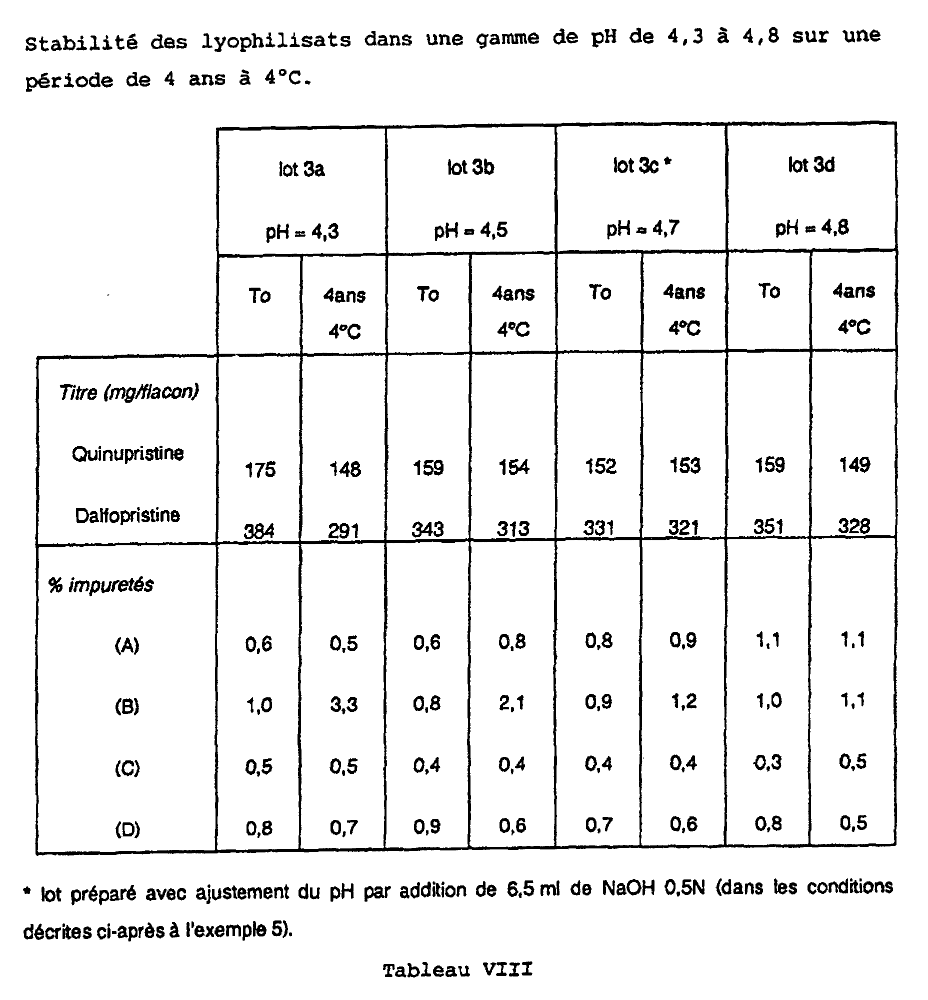

Stabilité des solutions destinées à la congélation, en cours de fabrication, dans les conditions de l'exemple 2 (Tableau IX) :

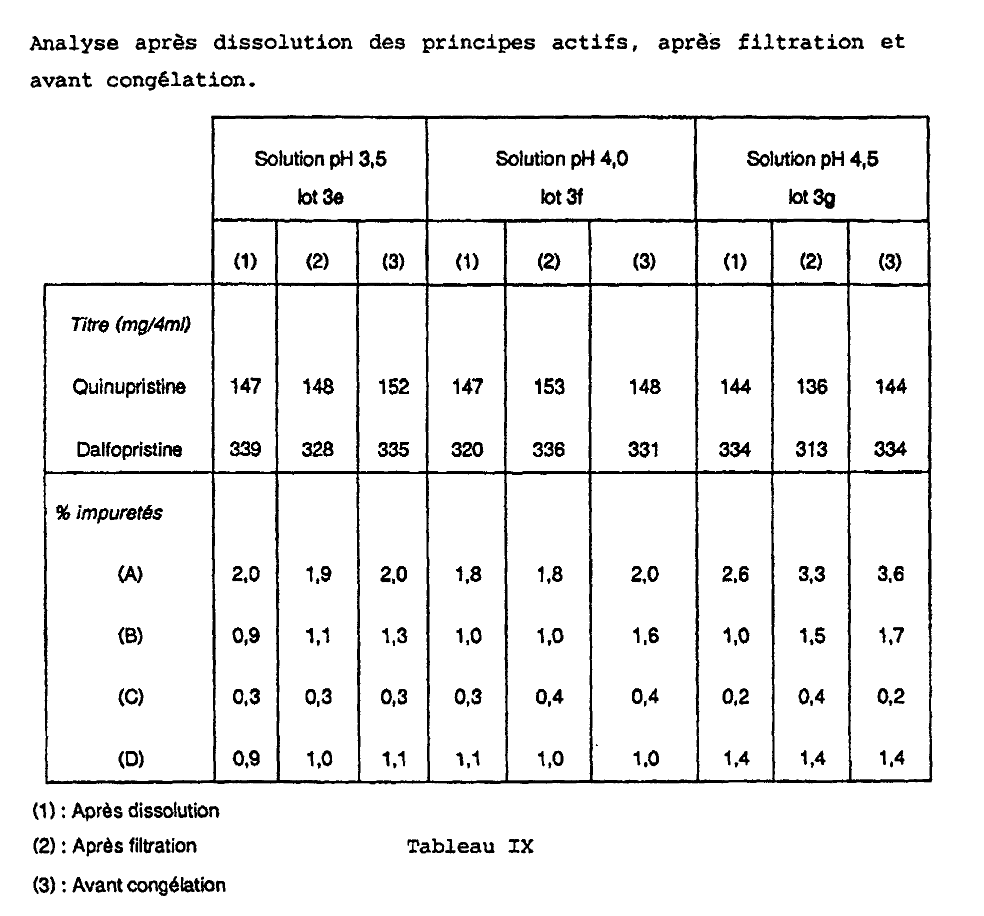

### Exemple 4

On prépare selon l'exemple 1, des solutions de quinupristine/dalfopristine (30/70) à un pH de 4,75 et à différentes concentrations comprises entre 125 mg/ml et 250mg/ml. Ces solutions sont utilisées pour l'obtention de lyophilisats à 500 mg. Les solutions les plus concentrées (200 et 250 mg/ml) sont plus lentes à mettre en oeuvre en raison du temps de mise en solution de la dalfopristine et de la durée d'ajustement du pH. L'étude montre que les profils de dégradation en cours de fabrication ne sont pas modifiés, seuls les temps de remise en solution des lyophilisats sont augmentés lorsque la concentration de la solution est augmentée (voir tableau X).

Profil de dégradation des solutions en cours de fabrication (après filtration et après lyophilisation)

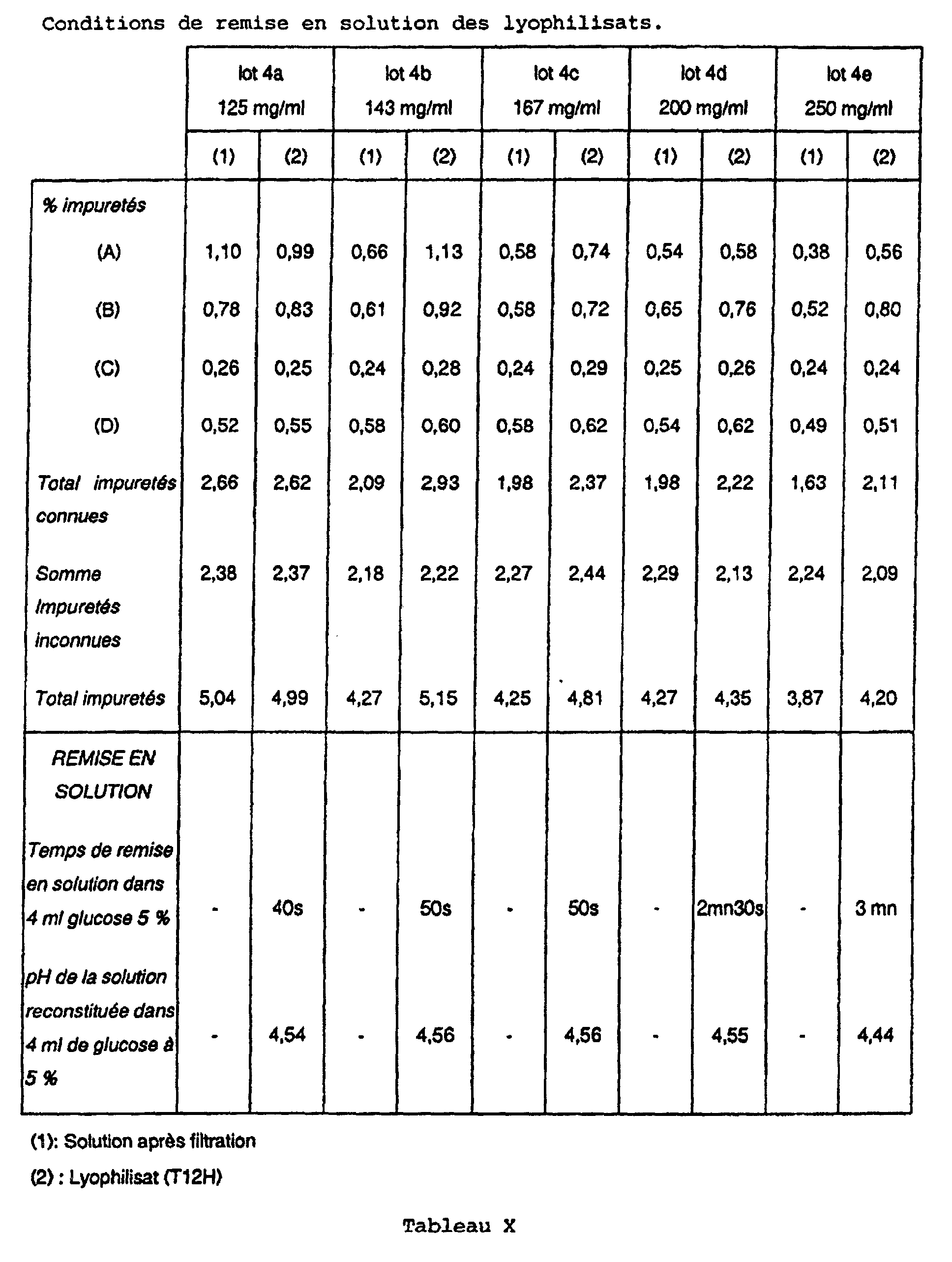

### Exemple 5

On prépare selon l'exemple 1, des solutions de quinupristine/dalfopristine (30/70) à 125 mg/ml pour lesquelles l'ajustement du pH à 4,75 est effectué par des quantités variables d'acide méthanesulfonique 1N (1 à 9 ml/l) et d'hydroxyde de sodium 0,5N (0 à 15 ml/l).

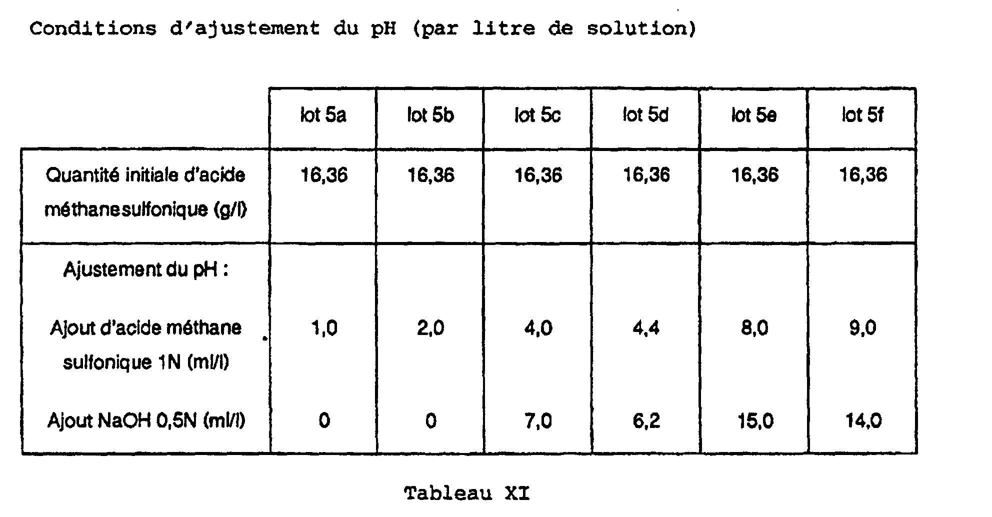

La stabilité des solutions fabriqués (étudiée à 3°C sur une période de 20 heures) ne montre pas de différence selon les conditions d'ajustement du pH

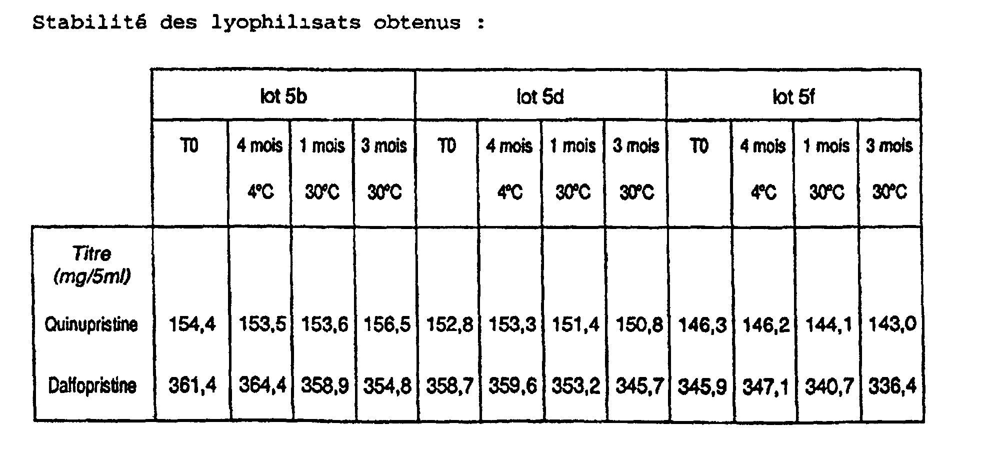

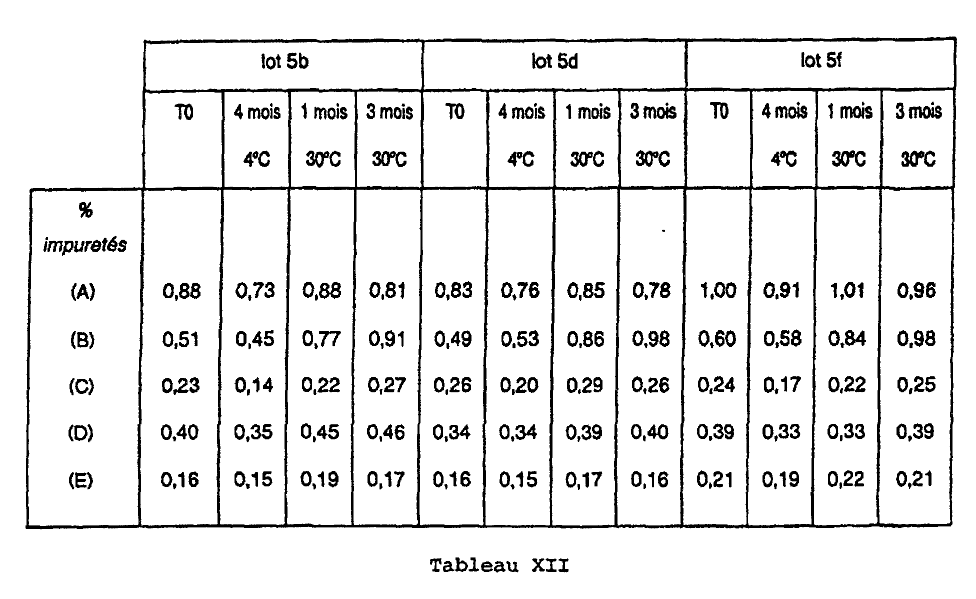

### Exemple 6

On prépare des solutions congelées de quinupristine/dalfopristine (30/70) salifiées par de l'acide méthanesulfonique ou de l'acide chlorhydrique, dans une gamme de concentration de 5 et 20 mg/ml, et à des pH de 3,5 et 5,0 et en présence d'agents isotonisants tels que le NaCl et le glucose. La stabilité des solutions stockées sous forme congelée est jugée satisfaisante.
(a) 800 g d'eau pour préparation injectable sont introduits dans une cuve de dissolution équipée d'une station de réfrigération. La solution est réfrigérée à une température comprise entre 0 et 6°C tout au long de la fabrication. On ajoute 98% de la quantité d'acide méthanesulfonique nécessaire à la dissolution et à l'ajustement du pH. 1,5 g de quinupristine sont introduits et dissous par agitation mécanique. 3,5 g de dalfopristine sont introduits et dissous par agitation mécanique. La solution est isotonisée avec du glucose. Le pH de la solution est ajusté à 5,0 par une solution 0,1N d'acide méthanesulfonique. La solution est complétée à 1 litre avec de l'eau pour préparation injectable. 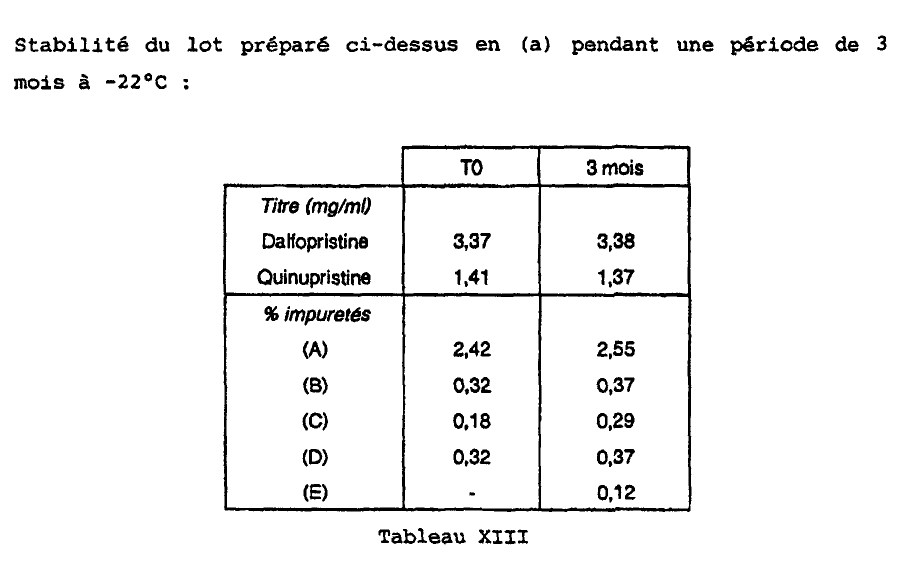
(b) On opère comme décrit ci-dessus, mais en introduisant 6 g de quinupristine et 14 g de dalfopristine. Le pH de la solution est ajusté à 3,5 par une solution 0,1N d'acide méthanesulfonique. La solution est complétée à 1 litre avec de l'eau pour préparation injectable. 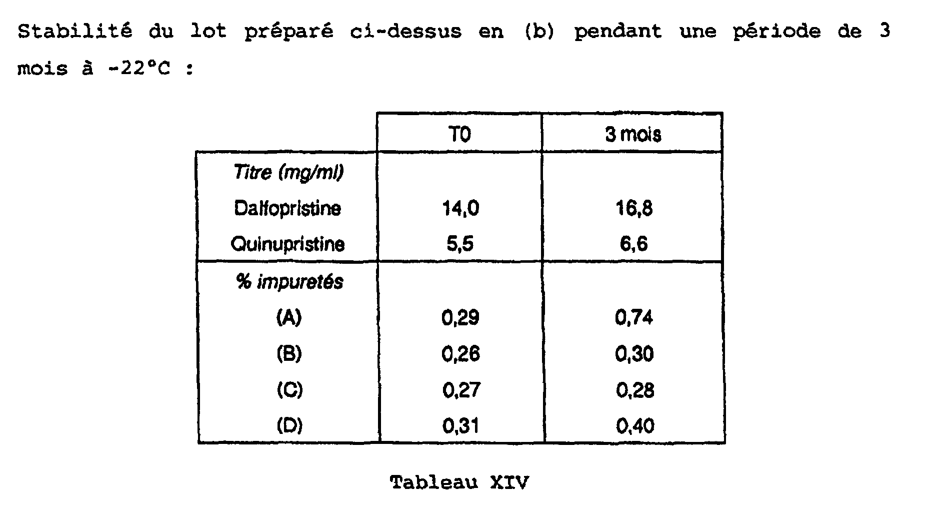
(c) On opère comme décrit ci-dessus en (a), mais en isotonisant la solution par du chlorure de sodium et en ajustant le pH à 3,5 par une solution 0,1N d'acide méthanesulfonique. La solution est complétée à 1 litre avec de l'eau pour préparation injectable. 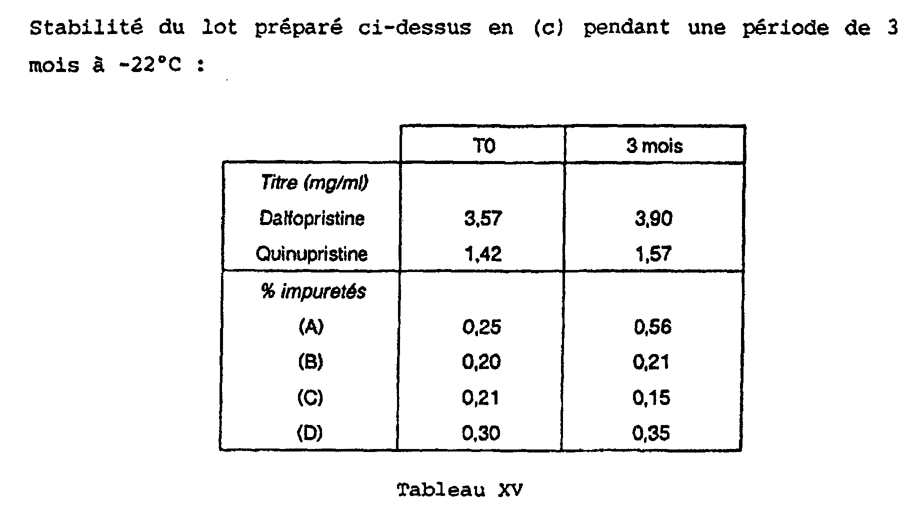
(d) On opère comme décrit ci-dessus en (c), mais en ajustant le pH à 5,0 par une solution 0,1N d'acide chlorhydrique. La solution est complétée à 1 litre avec de l'eau pour préparation injectable.

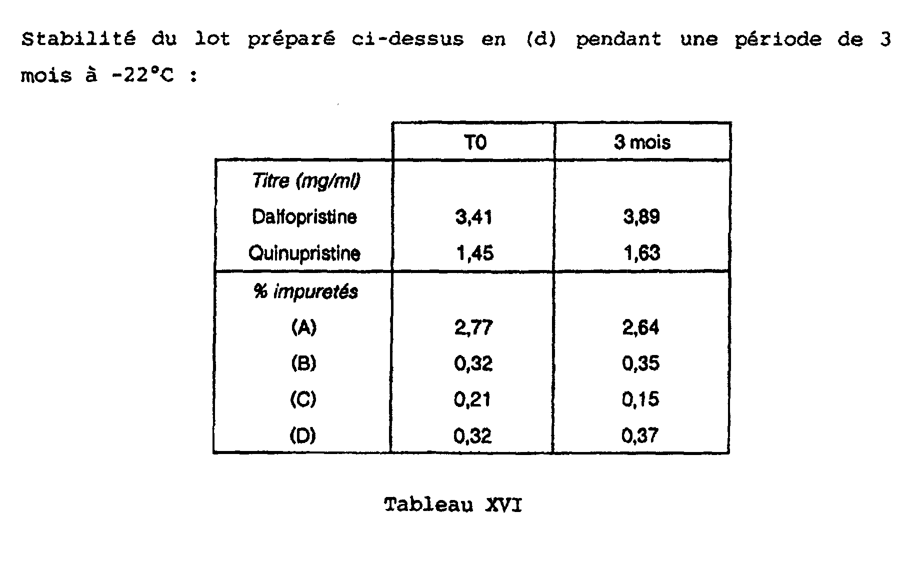

## Revendications

1. Composition pharmaceutique stabilisée, à base de l'association quinupristine / dalfopristine, **caractérisée en ce qu'**elle comprend de l'acide méthanesulfonique ou chlorhydrique, au moins en quantité stoechiométrique par rapport à la somme de la dalfopristine et de la quinupristine introduites et **en ce que** son pH est compris dans l'intervalle [3,5 ; 5].

2. Composition pharmaceutique stabilisée, selon la revendication 1, **caractérisée en ce qu'**elle peut être lyophilisée, congelée ou à l'état liquide.

3. Composition pharmaceutique stabilisée, selon la revendication 1 ou 2, **caractérisée en ce qu'**elle comprend
- la dalfopristine et la quinupristine ;
- l'acide méthanesulfonique ou chlorhydrique, au moins en quantité stoechiométrique par rapport à la dalfopristine et la quinupristine introduites ;
- le cas échéant un excès d'acide méthanesulfonique ou chlorhydrique destiné à ajuster le pH de la solution ainsi formée, dans l'intervalle [3,5 ; 5] ;
- éventuellement un agent isotonisant et/ou d'autres adjuvants pharmaceutiquement acceptables;
- une quantité d'eau convenable pour ajuster la concentration de la solution,
et **en ce que** l'eau est éliminée dans le cas d'un lyophilisat.

4. Composition pharmaceutique stabilisée, selon la revendication 3, **caractérisée en ce que** la concentration en principe actif quinupristine / dalfopristine est comprise entre 5 et 250 mg/ml et lorsqu'il s'agit d'un lyophilisat la proportion en principe actif est comprise entre 5 et 95 %.

5. Composition pharmaceutique stabilisée, selon la revendication 4, **caractérisée en ce que** la concentration en principe actif quinupristine / dalfopristine dans le lyophilisat est comprise entre 20 et 90 %.

6. Composition pharmaceutique stabilisée, selon l'une des revendications 1 à 4, **caractérisée en ce que** le pH est compris dans l'intervalle [3,5 ; 4,5].

7. Composition pharmaceutique stabilisée, selon la revendications 6, **caractérisée en ce que** le pH est 3,5.

8. Composition pharmaceutique stabilisée, selon l'une des revendications 1 à 4, **caractérisée en ce que** le pH est compris dans l'intervalle [4,5 ; 5].

9. Préparation d'une solution stabilisée prête à l'emploi à partir d'une composition pharmaceutique stabilisée, lyophilisée ou congelée, selon la revendication 2.

10. Procédé de préparation d'une composition pharmaceutique selon la revendication 1, **caractérisé en ce que** l'on dissout simultanément ou successivement la quinupristine, la dalfopristine, l'acide méthanesulfonique ou l'acide chlorhydrique dans de l'eau puis ajuste le pH dans l'intervalle [3,5 ; 5].

11. Procédé de préparation selon la revendication 10, **caractérisé en ce que** l'on dissout successivement la quinupristine puis la dalfopristine dans de l'eau acidifiée par l'acide méthanesulfonique ou l'acide chlorhydrique, puis ajuste le pH dans l'intervalle [3,5 ; 5].

12. Procédé selon la revendication 10 ou 11, pour la préparation d'une composition pharmaceutique selon la revendication 2, **caractérisé en ce que** le cas échéant la solution obtenue est ensuite lyophilisée et/ou congelée.

13. Utilisation de l'acide méthanesulfonique ou de l'acide chlorhydrique pour la préparation de compositions pharmaceutiques stabilisées selon l'une des revendications 1 à 8.

## Patentansprüche

1. Stabilisierte pharmazeutische Zusammensetzung auf der Basis der Assoziation Quinupristin/Dalfopristin, **dadurch gekennzeichnet, daß** sie Methansulfonsäure oder Chlorwasserstoffsäure mindestens in stöchiometrischer Menge umfaßt, bezogen auf die Summe von eingetragenem Dalfopristin und Quinupristin, und dadurch, daß ihr pH-Wert im Intervall zwischen [3,5 ; 5] liegt.

2. Stabilisierte pharmazeutische Zusammensetzung nach Anspruch 1, **dadurch gekennzeichnet, daß** sie lyophilisiert, eingefroren oder im flüssigen Zustand vorliegen kann.

3. Stabilisierte pharmazeutische Zusammensetzung nach Anspruch 1 oder 2, **dadurch gekennzeichnet, daß** sie umfaßt:
- Dalfopristin und Quinupristin;
- Methansulfonsäure oder Chlorwasserstoffsäure, mindestens in stöchiometrischer Menge, bezogen auf die eingetragenen Dalfopristin und Quinupristin;
- gegebenenfalls einen Überschuß von Methansulfonsäure oder Chlorwasserstoffsäure, der dazu vorgesehen ist, den pH-Wert der auf diese Weise gebildeten Lösung im Intervall zwischen [3,5 ; 5] einzustellen;
- gegebenenfalls ein isotonisches Mittel und/oder andere pharmazeutisch akzeptable Zusatzstoffe;
- eine geeignete Menge von Wasser, um die Konzentration der Lösung einzustellen;
und dadurch, daß das Wasser im Fall eines Lyophilisates entfernt ist.

4. Stabilisierte pharmazeutische Zusammensetzung nach Anspruch 3, **dadurch gekennzeichnet, daß** die Konzentration an Wirkstoff Quinupristin/Dalfopristin zwischen 5 und 250 mg/ml liegt, und wenn es sich um ein Lyophilisat handelt, das Verhältnis an Wirkstoff zwischen 5 und 95 % beträgt.

5. Stabilisierte pharmazeutische Zusammensetzung nach Anspruch 4, **dadurch gekennzeichnet, daß** die Konzentration an Wirkstoff Quinupristin/Dalfopristin in dem Lyophilisat zwischen 20 und 90 % beträgt.

6. Stabilisierte pharmazeutische Zusammensetzung nach einem der Ansprüche 1 bis 4, **dadurch gekennzeichnet, daß** der pH-Wert in dem Intervall [3/5 ; 4,5] liegt.

7. Stabilisierte pharmazeutische Zusammensetzung nach Anspruch 6, **dadurch gekennzeichnet, daß** der pH-Wert 3,5 beträgt.

8. Stabilisierte pharmazeutische Zusammensetzung nach einem der Ansprüche 1 bis 4, **dadurch gekennzeichnet, daß** der pH-Wert in dem Intervall [4,5 ; 5] liegt.

9. Herstellung einer stabilisierten, für den Gebrauch fertigen Lösung, ausgehend von einer stabilisierten, lyophilisierten oder eingefrorenen pharmazeutischen Zusammensetzung nach Anspruch 2.

10. Verfahren zur Herstellung einer pharmazeutischen Zusammensetzung nach Anspruch 1, **dadurch gekennzeichnet, daß** man gleichzeitig oder nacheinander das Quinupristin, das Dalfopristin, die Methansulfonsäure oder die Chlorwasserstoffsäure in Wasser auflöst und den pH-Wert im Intervall von [3,5 ; 5] einstellt.

11. Verfahren zur Herstellung nach Anspruch 10, **dadurch gekennzeichnet, daß** man nacheinander das Quinupristin und danach das Dalfopristin in Wasser auflöst, das mit Methansulfonsäure oder Chlorwasserstoffsäure angesäuert ist, und anschließend den pH-Wert im Intervall von [3,5 ; 5] einstellt.

12. Verfahren nach Anspruch 10 oder 11 zur Herstellung einer pharmazeutischen Zusammensetzung nach Anspruch 2, **dadurch gekennzeichnet, daß** die erhaltene Lösung anschließend gegebenenfalls lyophilisiert und/oder eingefroren wird.

13. Verwendung von Methansulfonsäure oder Chlorwasserstoffsäure für die Herstellung von stabilisierten pharmazeutischen Zusammensetzungen nach einem der Ansprüche 1 bis 8.

## Claims

1. Stabilized pharmaceutical composition based on the quinupristine/dalfopristine combination, **characterized in that** it comprises methanesulphonic or hydrochloric acid, in an at least stoichiometric amount with respect to the sum of the dalfopristine and of the quinupristine introduced, and **in that** its pH is within the range [3.5 ; 5].

2. Stabilized pharmaceutical composition according to Claim 1, **characterized in that** it can be lyophilized, frozen or in the liquid state.

3. Stabilized pharmaceutical composition according to Claim 1 or 2, **characterized in that** it comprises:
- dalfopristine and guinupristine;
- methanesulphonic or hydrochloric acid, in an at least stoichiometric amount with respect to the dalfopristine and the quinupristine introduced;
- if appropriate, an excess of methanesulphonic or hydrochloric acid intended to adjust the pH of the solution thus formed in the range [3.5 ; 5];
- optionally, an isotonizing agent and/or other pharmaceutically acceptable adjuvants;
- an amount of water suitable for adjusting the concentration of the solution,
and **in that** the water is removed in the case of a lyophilisate.

4. Stabilized pharmaceutical composition according to Claim 3, **characterized in that** the concentration of quinupristine/dalfopristine active principle is between 5 and 250 mg/nl and, when it is lyophilisate, the proportion of active principle is between 5 and 95%.

5. Stabilized pharmaceutical composition according to Claim 4, **characterized in that** the concentration of quinupristine/dalfopristine active principle in the lyophilisate is between 20 and 90%.

6. Stabilized pharmaceutical composition according to one of Claims 1 to 4, **characterized in that** the pH is within the range [3.5 ; 4.5].

7. Stabilized pharmaceutical composition according to Claim 6, **characterized in that** the pH is 3.5.

8. Stabilized pharmaceutical composition according to one of Claims 1 to 4, **characterized in that** the pH is within the range [4.5 ; 5].

9. Preparation of a stabilized ready-for-use solution from a lyophilized or frozen stabilized pharmaceutical composition according to Claim 2.

10. process for the preparation of a pharmaceutical composition according to Claim 1, **characterized in that** quinupristine, dalfopristine, methanesulphonic acid or hydrochloric acid are simultaneously or successively dissolved in water and then the pH is adjusted in the range [3.5 ; 5].

11. Preparation process according to Claim 10, **characterized in that** quinupristine and then dalfopristine are dissolved successively in water acidified by methanesulphonic acid or hydrochloric acid and then the pH is adjusted in the range [3.5 ; 5].

12. Process according to Claim 10 or 11 for the preparation of a pharmaceutical composition according to Claim 2, **characterized in that**, if appropriate, the solution obtained is subsequently lyophilized and/or frozen.

13. Use of methanesulphonic acid or of hydrochloric acid for the preparation of stabilized pharmaceutical. compositions according to one of Claims 1 to 8.
